# EUROPÄISCHE PATENTANMELDUNG

(11) **EP 1 470 814 A1**
(43) Veröffentlichungstag der Anmeldung: **27.10.2004**
(21) Anmeldenummer: 04017279.3
(22) Anmeldetag: 29.01.2002
(51) Int. Cl.: A61K 9/14, A61K 9/16, A61K 9/72, B01F 13/00, B01J 19/00, A61K 9/00, B01D 9/00, B01F 5/06

(54) **Vorrichtung zur Durchführung eines Verfahrens zur kontinuierlichen Herstellung inhalierfähiger Arzneistoffe und nach diesem Verfahren hergestellter Arzneistoff**

(30) Priorität: 21.04.2001 DE 10119718
(62) Teilanmeldung aus: 02722039.1
(71) Anmelder: Boehringer Ingelheim Pharma GmbH & Co.KG, 55218 Ingelheim am Rhein (DE)
(72) Erfinder: Zierenberg, Bernd, 55411 Bingen am Rhein (DE); Schiewe, Jörg, 55129 Mainz (DE)
(74) Vertreter: Fuchs Mehler Weiss & Fritzsche

(57) **Zusammenfassung**

Die Erfindung betrifft einen Mikroreaktor zur Durchführung eines Verfahrens zur kontinuierlichen Herstellung inhalierfähiger Arzneistoffe, bei dem eine Arzneistoff-Lösung (11) eingesetzt wird, die Lösung mittels eines Segmenters und eines Transportmediums (22) segmentiert wird, der Kristallisationsprozess in einer Verweilerstrecke (3) mittels Aufprägen einer definierten Temperatur eingeleitet und geführt wird, wobei zunächst mittels einer sprunghaften Temperaturverringerung der Keimbildungsprozess in der Art eingeleitet wird, dass die Lösung bzgl. der Temperatur T und der Konzentration C des in ihr gelösten Stoffes einen übersättigten, metastabilen oder labilen Zustand annimmt, und anschließend das Kristallwachstum durch gezielte Kühlung beeinflusst wird, und Kristallpartikel von den übrigen Phasen nach Durchlauf der Verweilerstrecke in einem Abscheider abgeschieden werden. Der Mikroreaktor zeichnet sich dadurch aus, dass
- die Dimensionen des Mikromischers zur Aufteilung der zugeführten, zu mischenden Fluide im Bereich von 10µm bis 1 mm, vorzugsweise zwischen 25µm bis 200 µm, liegen,
- die Dimensionen der Kanäle des Segmenters im Bereich von 0,1 bis 5 mm, vorzugsweise im Bereich zwischen 0,2 mm und 5 mm liegen, und
- die Verweilerstrecke schlauch-, rohr- oder kanalförmig ausgebildet ist mit Durchmessern ihrer Kanäle im Bereich von 0,5 bis 10 mm, vorzugsweise 1 mm bis 2 mm, und eine Länge aufweist zwischen 10 cm und 200 m, vorzugsweise zwischen 1 m und 25 m.

## Beschreibung

Die Erfindung betrifft eine Vorrichtung zur Durchführung eines Verfahrens zur kontinuierlichen Herstellung inhalierfähiger Arzneistoffe und einen Arzneistoff, der die an inhalierfähige Arzneistoffe gestellten Anforderungen erfüllt.

Im Rahmen der Erfindung ist unter dem Begriff "Arzneistoff" der wirksame Bestandteil eines Arzneimittels zu verstehen, der üblicherweise auch als Pharmakon oder Wirkstoff bezeichnet wird.

Inhalativa erfordern eine bestimmte Erscheinungsform des Arzneistoffes. Zum Einsatz kommen in der Regel mikronisierte Arznei- bzw. Wirkstoffe in fester Form. Um die Inhalierfähigkeit des Arzneistoffes zu gewährleisten, werden hohe Anforderungen an die Teilchengröße, die Teilchengrößenverteilung, die Morphologie, die Stabilität und das Fließverhalten gestellt.

In der Regel gelangt nicht die gesamte inhalativ verabreichte Dosis des Arzneistoffes in die Lunge, sondern nur ein Teil dieser Dosis. Maßgeblichen Einfluss auf den Anteil des Arzneistoffes, der tatsächlich in die Lunge gelangt, hat die Teilchengröße. Aus diesem Grunde werden Teilchen bevorzugt, die einen Durchmesser kleiner 20µm, vorzugsweise kleiner 5µm und größer 0,3µm, aufweisen. Der Durchmesser des Teilchens sollte sich im angegebenen Fenster befinden und darüber hinaus eine möglichst enge Größenverteilung aufweisen. Größere Teilchen werden beim Einatmen bereits in den oberen Luftwegen abgeschieden, wohingegen kleinere Teilchen nicht in der Lunge deponiert werden und diese beim Ausatmen wieder verlassen.

Unter Teilchendurchmesser im Rahmen der vorliegenden Erfindung ist der aerodynamische Partikeldurchmesser zu verstehen, wobei dieser definiert ist als Äquivalentdurchmesser einer Kugel der Dichte von 1 g/cm³, die die gleiche Sentimedationsgeschwindigkeit in Luft besitzt, wie das untersuchte Teilchen.

Des Weiteren werden hohe Anforderungen an die physikalische Stabilität der mikronisierten Arzneistoffteilchen gestellt. Die Teilchen sollten bei Umgebungsbedingungen vorzugsweise in der stabilen Kristallform vorliegen, um Agglomeration durch Phasenumwandung zu verhindern. Die Stabilität der Arzneistoffteilchen hat somit indirekten Einfluss auf die tatsächlich in die Lunge gelangte Arzneistoffmenge. Aus diesem Grunde werden hohe Anforderungen an die Stabilität des Arzneistoffes gestellt, um eine dauerhaft gleich bleibende Qualität, insbesondere eine zeitlich konstante Teilchengröße bzw. Größenverteilung, des Arzneistoffes zu gewährleisten. Gerade im Bereich der Pharmazie und der Verwendung von Arzneistoffen ist dieses Qualitätsmerkmal unerlässlich, weil die Wirkung des Arzneistoffes von der in die Lunge gelangten Dosis und somit, wie oben beschrieben, von der Teilchengröße und ihrer Größenverteilung abhängt.

Ähnliches gilt für die Morphologie der mikronisierten Teilchen, da die Beschaffenheit der Teilchenoberfläche direkten Einfluss auf die Neigung der Teilchen zur Agglomeration und somit indirekten Einfluss auf die Teilchengröße selbst bzw. die Haltbarkeit des Arzneistoffes hat.

Dem mikronisierten Arzneistoff können Hilfsstoffe zugesetzt werden, mit denen die physiko-chemischen Eigenschaften eines Arzneimittels eingestellt werden, wobei diese die qualitätsbestimmenden Parameter, wie Bioverfügbarkeit, Wirksamkeit und Haltbarkeit in gewünschter Weise beeinflussen.

Neben der Teilchengröße und Größenverteilung des mikronisierten Arzneistoffs können Art, Teilchengröße und Megenverhältnis der zugesetzten Hilfsstoffe in entscheidender Weise die Arzneistoff-Dosis beeinflussen, die in die Lunge gelangt.

Herkömmliche Verfahren zur Herstellung inhalierfähiger Arzneistoffe sind in der Regel, eine grob strukturierte Betrachtungsweise vorausgesetzt, zweistufig, wobei in einer ersten Stufe der Arzneistoff in fester, üblicherweise kristallinen Form hergestellt und dieser in einer zweiten Stufe im Rahmen eines Zerkleinerungsprozesses in mikronisierte Teilchen transformiert wird. Nach dem Stand der Technik kommen für den Zerkleinerungsprozess Mahlprozesse zum Einsatz, wobei insbesondere Luftstrahlmahlen eine große Bedeutung erlangt hat, da es ökonomisch arbeitet, für eine Vielzahl von Substanzen anwendbar ist und eine einfache Abtrennung der gewünschten Teilchenfraktionen durch einen nachgeschalteten Zyklon-Abscheider erlaubt.

Nachteilig an dem nach dem Stand der Technik verwendeten Luftstrahlmahlen ist, dass die Feststoffteilchen prinzipbedingt einer erheblichen Krafteinwirkung während des Mahlprozesses ausgesetzt sind. Diese Krafteinwirkung induziert eine beträchtliche lokale Erwärmung und führt darüber hinaus zur Bildung amorpher Anteile. Aufgrund der lokalen Erwärmung eignet sich das Luftstrahlmahlen bzw. das Mahlen als Zerkleinerungsprozeß generell nicht für niedrigschmelzende, thermisch labile oder denaturierbare Stoffe.

Darüber hinaus wird bei der Lagerung strahlgemahlener Arzneistoffe häufig eine Agglomeration beobachtet, da die durch den Mahlprozess entstandenen amorphen Anteile rekristallisieren.

Vor diesem Hintergrund ist es die Aufgabe der vorliegenden Erfindung, eine Vorrichtung zur Durchführung eines Verfahrens zur kontinuierlichen Herstellung inhalierfähiger Arzneistoffe, bei dem die Einhaltung der genannten Anforderungen an Arzneistoffe sichergestellt ist und darüber hinaus die Nachteile der nach dem Stand der Technik verwendeten Verfahren vermieden werden, bereitzustellen.

Eine weitere Teilaufgabe der vorliegenden Erfindung ist es, einen Arzneistoff bereitzustellen, der die Anforderungen an einen inhalierfähigen Arzneistoff, insbesondere die Anforderungen an die Teilchengröße, die Größenverteilung, die Morphologie und die Stabilität erfüllt.

Gelöst wird die Aufgabe durch einen Mikroreaktor zur Durchführung eines Verfahrens zur kontinuierlichen Herstellung inhalierfähiger Arzneistoffe, bei dem eine Arzneistoff-Lösung eingesetzt wird, die Lösung mittels eines Segmenters und eines Transportmediums segmentiert wird, der Kristallisationsprozess in einer Verweilerstrecke mittels Aufprägen einer definierten Temperatur eingeleitet und geführt wird, wobei zunächst mittels einer sprunghaften Temperaturverringerung der Keimbildungsprozess in der Art eingeleitet wird, dass die Lösung bzgl. der Temperatur T und der Konzentration C des in ihr gelösten Stoffes einen übersättigten, metastabilen oder labilen Zustand annimmt, und anschließend das Kristallwachstum durch gezielte Kühlung beeinflusst wird, und Kristallpartikel von den übrigen Phasen nach Durchlauf der Verweilerstrecke in einem Abscheider abgeschieden werden, dadurch gekennzeichnet, dass
- die Dimensionen des Mikromischers zur Aufteilung der zugeführten, zu mischenden Fluide im Bereich von 10µm bis 1 mm, vorzugsweise zwischen 25µm bis 200 µm, liegen,
- die Dimensionen der Kanäle des Segmenters im Bereich von 0,1 bis 5 mm, vorzugsweise im Bereich zwischen 0,2 mm und 5 mm liegen, und
- die Verweilerstrecke schlauch-, rohr- oder kanalförmig ausgebildet ist mit Durchmessern ihrer Kanäle im Bereich von 0,5 bis 10 mm, vorzugsweise 1 mm bis 2 mm, und eine Länge aufweist zwischen 10 cm und 200 m, vorzugsweise zwischen 1 m und 25 m.

Der geringe Durchmesser der Verweilerstrecke bzw. das große Oberflächen-Nolumenverhältnis impliziert eine geringe in der Verweilerstrecke geführte Lösungsmenge, welche nur begrenzt Wärme speichern kann. Aus diesem Grunde und aufgrund des Oberflächen-/Volumenverhältnisses kann der in der Verweilerstrecke befindlichen Lösung von außen in sehr kurzer Zeit eine definierte Temperatur aufgeprägt werden, wodurch eine schnelle Temperaturführung ermöglicht wird. Ebenfalls aufgrund der geringen Abmessungen werden nur sehr geringe Temperaturgradienten in der Lösung beobachtet und somit kann von einer weitestgehend homogenen Temperaturverteilung ausgegangen werden. Dies ist insofern hervorzuheben, da es für die Effektivität der Kristallisation wichtig ist, dass die lokalen Bedingungen in der Verweilerströmung nicht variieren und sich im gesamten Lösungsvolumen die gewünschten Parameter einstellen.

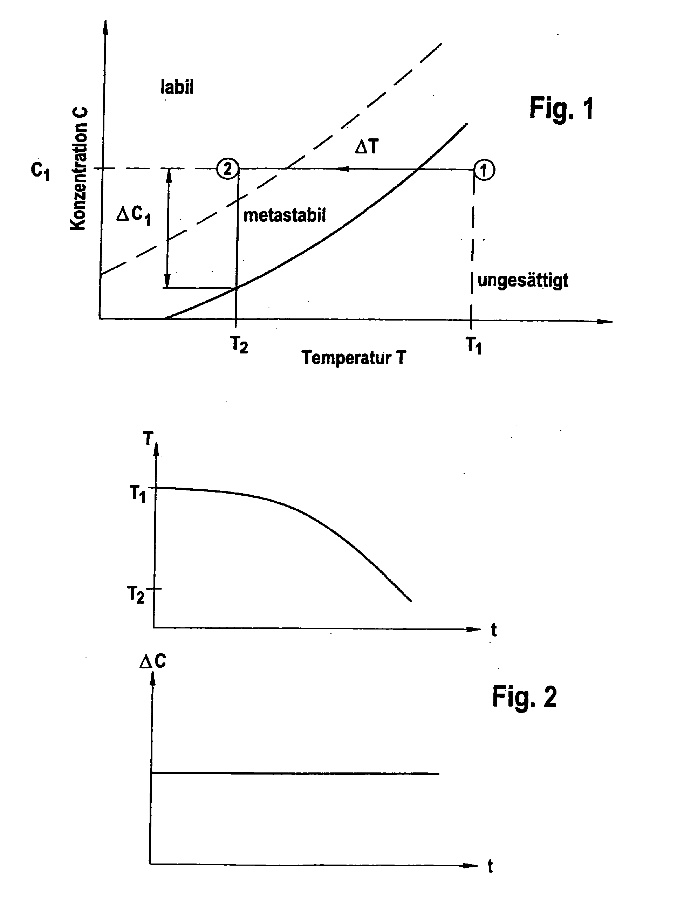

Bei dem Verfahren treten keine unerwünschten Temperaturschwankungen auf, da die Temperatur in der Lösung von außen gezielt und schnell eingestellt werden kann und damit der Keimbildungsprozess und das Kristallwachstum exakt gesteuert werden können. Würde die Lösung bei Verwendung von herkömmlichen Kristallisatoren entsprechend Figur 1 von Zustand 1 in Zustand 2 so überführt, dass eine hohe Übersättigung eintritt, käme es zu nicht beeinflussbaren Temperaturschwankungen, da der in Zustand 2 ablaufende Keimbildungsprozess und die bei hoher Keimzahl entstehende, erhebliche Kristallisationswärme den Zustand hin zu höheren Temperaturen verschieben würde und ein Gegensteuern in Form eines Kühlens aufgrund der konstruktiven Abmessungen herkömmlicher Kristallisatoren zu periodischen Temperaturschwankungen führen würde.

Nach Durchlaufen der Verweilerstrecke wird das Produktgemisch einem Abscheider zugeführt, in welchem die erzeugten Kristallpartikel von den übrigen Phasen getrennt werden, so dass am Ende des Herstellungsverfahrens, nach Durchlaufen des Abscheiders der Arzneistoff mit den gewünschten Eigenschaften vorliegt.

Vorteilhaft sind Verfahren, bei denen die Arzneistoff-Lösung unter Auflösen des festen Arzneistoffes in einem Lösungsmittel zur Bildung einer solchen Arzneistoff-Lösung bereitgestellt wird.

Dabei wird der feste Arzneistoff in einem Lösungsmittel, in dem er sich bei vorgegebener Temperatur vollständig löst, zur Bildung einer Arzneistoff-Lösung aufgelöst.

Vorteilhaft sind Verfahren, bei denen
- die Segmentierung der Lösung mittels eines Segmenters und eines Transportmediums unter Ausbildung von Plug-Flow-Bedingungen erfolgt.

Wesentlicher Vorteil dieser Ausführungsform ist, dass die aus dem Segmenter austretende Zweiphasenströmung in der rohrförmigen Verweilerstrecke, die sie im Anschluss durchläuft, kein parabolisches Geschwindigkeitsprofil (Hagen-Poiseulliesches-Gesetz) ausbildet, sondern sich ein über die Zeit gemitteltes Rechteckprofil einstellt. Dies unterscheidet das Verfahren von den in herkömmlichen Kristallisatoren ablaufenden Verfahren, bei denen sich ein parabolisches Profil einstellt, so dass sich die Geschwindigkeit in den Rohrrandbereichen verringert und schließlich direkt an der Wand zu Null wird.

Als Folge dieses parabolischen Geschwindigkeitsprofils kommt es an der Rohrinnenwand der herkömmlichen Kristallisatoren zu Ablagerungen insbesondere von weiter wachsenden Kristallen, wodurch die Kristallisatoren letztendlich verstopfen bzw. sich zusetzen.

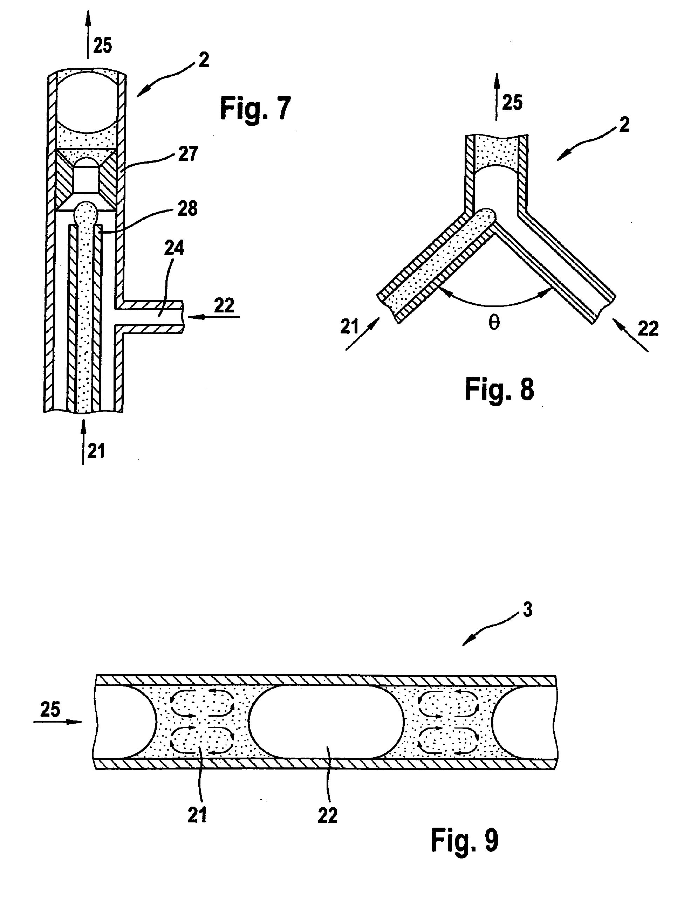

Bezüglich der Plug-Flow-Bedingungen sei auf Figur 9 verwiesen, in dem die in den Volumensegmenten vorliegenden Strömungen bzw. Strömungsrichtungen eingezeichnet sind. Die Strömungsvorgänge in den Segmenten unterstützen die Homogenisierung der Lösung und wirken der Ausbildung von Konzentrationsunterschieden im Inneren und an der Wand entgegen. Des weiteren wird ein Verstopfen durch Kristallablagerung an den Innenwänden der Verweilerstrecke vermieden.

Vorteilhaft sind Ausführungsformen des Verfahrens, bei denen
- die Arzneistoff-Lösung in einem Mischer mit einem Fällungsmittel zu einer homogenen Fällungslösung vermengt wird und diese Fällungslösung das weitere Verfahren durchläuft.

Das Fällungsmittel wird der Arzneistoff-Lösung zugemischt, um die Löslichkeit des Arzneistoffs in der Mischung bei gegebener Temperatur zu vermindern, so dass Festkörperpartikel gebildet werden. Diese Arzneistoff-Lösung wird dabei in einem dem Segmenter vorgeschalteten Verfahrensschritt zur Herstellung einer möglichst homogenen Fällungslösung mit einem Fällungsmittel in einem Mischer durchmengt. Mikromischer eignen sich hierzu besonders gut, da in ihnen Masse- und Wärmetransportvorgänge schnell und effizient ablaufen. Unter Mikromischer im Rahmen der vorliegenden Erfindung ist eine Struktur zu verstehen, welche Dimensionen im Bereich von 10µm bis 1 mm, vorzugsweise zwischen 25µm bis 200µm, aufweist.

Zur Herstellung von mikronisierten und inhalierfähigen Arzneistoffen ist eine Vermischung der Arzneistoff-Lösung und dem Fällungsmittel zu einer Fällungslösung von möglichst hoher Homogenität erforderlich. Ein Mikromischer mit seinen filigranen Strukturen eignet sich hierfür in besonderem Maße. Bei ihm werden die beiden Fluide, einerseits die Arzneistoff-Lösung und anderseits das Fällungsmittel, nach Eintritt in den Mischer mittels einer Mikrostruktur in Einzelströme aufgeteilt. Die Einzelströme sind beispielsweise lamellenförmig und werden mit Hilfe von in der Mikrostruktur angeordneten Kanälen in der Art geschichtet, dass ein System aus dünnen Fluidlamellen entsteht, bei welchem abwechselnd eine Fluidlamelle der Arzneistoff-Lösung einer Fluidlamelle des Fällungsmittels benachbart ist. Dabei beträgt die Lamellendichte im Mikromischer 10 bis 1000, vorzugsweise 20 bis 500 pro cm. Das so geschichtete, aus einer Vielzahl von Lamellen bestehende Fluidsystem wird einer Mischkammer zugeführt, in der eine Vermischung durch Diffusion erfolgt. Die Mischung nach dem Prinzip der Diffusion kann nur in akzeptabel kurzen Zeiten vollzogen werden, wenn die Strukturen des Mischers und damit die Lamellendicke der Einzelströme genügend klein sind (vorzugsweise 10 bis 200 µm).

Die Dicke der Fluidlamellen bestimmt in entscheidender Weise die Zeit, die zum Ausgleich der Konzentrationsunterschiede durch diffusive Vermischung benötigt wird. Liegt die Lamellendicke im Bereich einiger zehn Mikrometer, kann eine komplette Vermischung und damit eine Homogenität der Fällungslösung im gesamten Mischvolumen bereits in einer Zeit unter einer Sekunde realisiert werden.

Der Mikromischer ist vorzugsweise in der Art ausgeführt, dass er auf einfache Weise beheizt und/oder gekühlt werden kann. Durchflussgeschwindigkeit und Temperatur im Mikromischer werden im Hinblick auf den Keimbildungsprozess so gewählt, dass eine Keimbildung im Mischer nicht stattfindet.

An dieser Stelle sei darauf hingewiesen, dass eine Vielzahl von Vorrichtungen zum Heizen und Kühlen der einzelnen, beschriebenen Bauelemente des Mikroreaktors verwendet werden können. Insbesondere sind dies Draht-Widerstandsheizungen, elektrische Heizfolien, Peltier-Elemente sowie Heiz- und/oder Kühlvorrichtungen, die mit einem temperierten Fluid wie beispielsweise Wasser, Öl, Luft, Stickstoff und dergleichen arbeiten. Daneben können auch Infrarot-Strahlung und Mikrowellenheizungen eingesetzt werden.

Vorteilhaft sind auch Ausführungsformen des Mikroreaktors, bei denen auf der Basis einer geätzten Platte, die eine elektrische Heizung aufweist, ein Mikroreaktor in diese Platte implementiert ist.

Vorteilhaft sind Ausführungsformen des Verfahrens, bei denen submikrometergroße Festkörperpartikel zur Keimbildung im Segmenter eingetragen werden, wobei vorzugsweise
- der Eintrag der submikrometergroßen Festkörperpartikel bei der Verwendung von Luft als Transportmedium vorzugsweise durch Zugabe eines Hilfsstoffes oder des Arzneistoffes als Staubaerosol in den Luftstrom erfolgt, und
- bei Verwendung eines flüssigen Transportmediums submikrometergroße Festkörperpartikel in Form von Hilfsstoff- oder Arzneistoffpartikeln dem Transportmedium zugesetzt werden, oder
- bei Verwendung eines flüssigen Transportmediums submikrometergroße Festkörperpartikel in Form von Hilfsstoff-Kolloiden dem Transportmedium zugesetzt werden.

Durch Eintrag von submikrometergroßen Festkörperpartikeln lässt sich der Kristallisierungsprozess besser kontrollieren. Durch den Eintrag von Kristallkeimen kann der Arbeitsbereich auch auf den metastabilen Bereich (bei geringeren Übersättigungsverhältnissen) erweitert werden. Bei den drei genannten Varianten des Feststoffpartikeleintrages werden bei der im Segmenter erzeugten Zweiphasenströmung als Kristallisationskeime dienende Feststoffpartikel von außen, d.h. vom Transportmedium, in die segmentierte Lösung eingetragen.

Vorteilhaft sind Ausführungsformen des Verfahrens, bei dem submikrometergroße Festkörperpartikel zur Keimbildung im Mischer zugegeben werden, wobei vorzugsweise die Zugabe der submikrometergroßen Festkörperpartikel durch Verwendung eines Fällungsmittels, welches Kolloidpartikel enthält, erfolgt.

Vorteilhaft sind Ausführungsformen des Verfahrens, bei denen die Temperaturführung in der Verweilerstrecke in der Art erfolgt, dass eine im wesentlichen konstante Übersättigung ΔC₁ in der Lösung vorliegt. Dies trägt zur Lösung der erfindungsgemäßen Aufgabe bei, sehr kleine Teilchen bzw. Kristalle von geringem Durchmesser zu bilden. Figur 2 zeigt wie die Temperatur T(t) zu führen ist, um bei einem parabolischen Verlauf der Konzentration (Figur 1) und unter der Annahme eines diffusionskontrollierten Kristallwachstums eine konstante Übersättigung über der Zeit zu erhalten (ebenfalls Figur 2).

Für die Herstellung entsprechender Mengen des inhalierfähigen Arzneistoffes kann es erforderlich werden, mehrere Mikroreaktoren einzusetzen. Dabei werden beispielsweise 10 bis 100 derartiger Mikroreaktoren zu einem Verbund zusammengeschlossen, wobei diese unabhängig oder abhängig voneinander parallel betrieben werden. Mittels einer derartigen Batterie können Produktionsmengen von 0,5 bis 2 kg pro Tag realisiert werden.

Innerhalb einer solchen Batterie besteht die Möglichkeit der Verwendung nur einer Heiz- bzw. Kühlvorrichtung, die alle funktionell ähnlichen Einzelvorrichtungen heizt bzw. kühlt.

Der Mikroreaktor muss bedingt durch seine Mikrostrukturen selbst nicht unbedingt kleine Abmessungen aufweisen. Es kann sich bei dem Mikroreaktor vielmehr auch um eine technische Apparatur handeln, in die funktionale Miktrostrukturen der beschriebenen Art implementiert werden.

Die Teilaufgabe der vorliegenden Erfindung hinsichtlich des Bereitstellens eines inhalierfähiger Arzneistoff, der die Anforderungen an einen inhalierfähigen Arzneistoff erfüllt, wird gelöst durch einen Arzneistoff mit einem aerodynamischen Durchmesser kleiner 20µm, vorzugsweise kleiner 5µm und größer 0,3µm, der dadurch gekennzeichnet ist, dass er mittels der erfindungsgemäßen Vorrichtung hergestellt wird.

Die Erfindung wird anhand verschiedener Ausführungsbeispiele gemäß den Zeichnungsfiguren näher erläutert. Hierbei zeigt:
- Fig. 1: das Konzentrations-Temperatur-Diagramm der in die Verweilerstrecke eintretenden Lösung,
- Fig. 2: die Temperaturführung T(t) einer Ausführungsform des erfindungsgemäßen Verfahrens zur Einstellung einer konstanten Übersättigung Δ C (t),
- Fig. 3: schematisch eine Ausführungsform eines Mikroreaktors zur Durchführung des erfindungsgemäßen Verfahrens,
- Fig 4: einen Mikromischer des Mikroreaktors gemäß einer ersten Ausführungsform im Querschnitt,
- Fig. 4a: eine Vergrößerung der Strukturen der Kanäle des in Figur 4 dargestellten Mikromischers,
- Fig. 5: einen Segmenter des Mikroreaktors gemäß einer ersten Ausführungsform im Querschnitt,
- Fig. 6: einen Mikromischer des Mikroreaktors gemäß einer zweiten Ausführungsform mit integriertem Segmenter im Querschnitt,
- Fig. 7: einen Segmenter des Mikroreaktors gemäß einer dritten Ausführungsform im Querschnitt,
- Fig. 8: einen Segmenter des Mikroreaktors gemäß einer vierten Ausführungsform im Querschnitt,
- Fig. 9: die Verweilerstrecke einer Ausführungsform eines Mikroreaktors im Querschnitt mit der in der Verweilerstrecke geführten Zweiphasenströmung, und
- Fig. 10: eine Verweilerstrecke einer Ausführungsform eines Mikroreaktors mit den Temperaturverläufen über die Länge der Verweilerstrecke und über die Zeit.

Die Figuren 1 und 2 wurden bereits oben beschrieben.

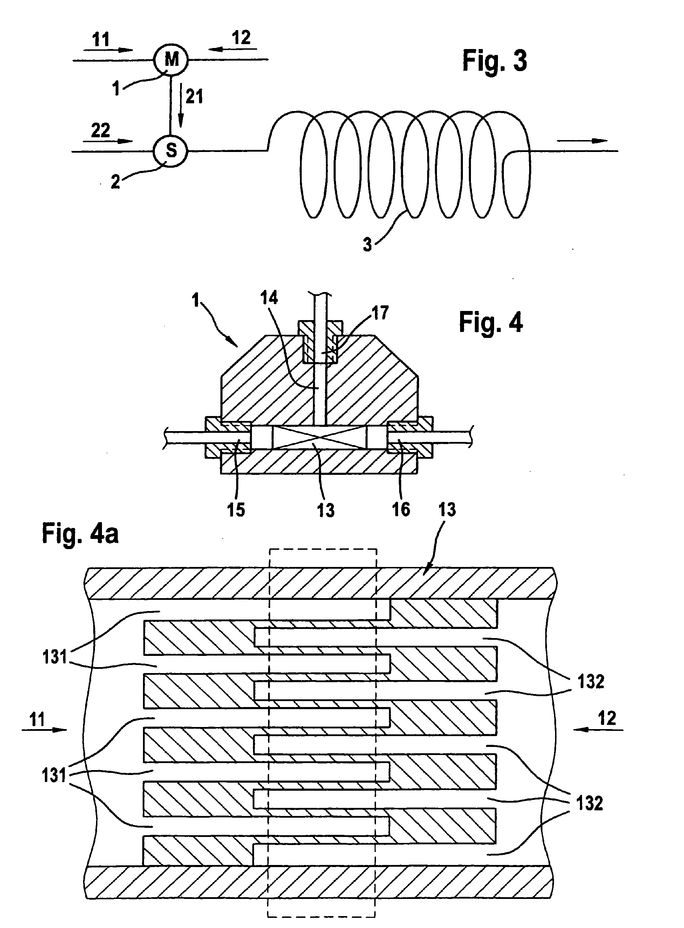

Figur 3 zeigt schematisch den Aufbau eines Mikroreaktors zur Durchführung einer ersten Variante des Verfahrens. Der Mikroreaktor besteht aus einem Mikromischer 1, einem Segmenter 2 und einer Verweilerstrecke 3. Dabei wird zunächst die Arzneistoff-Lösung 11 mit einem Fällungsmittel 12 im Mikromischer 1 zu einer möglichst homogenen Fällungslösung 21 vermischt. Diese Fällungslösung 21 wird dem Segmenter 2 zugeführt und mit Hilfe eines Transportmediums 22 segmentiert. Die nach Durchlaufen des Segmenters vorliegende Zweiphasenströmung wird der Verweilerstrecke 3 zugeführt, in der die Kristallisation erfolgt.

Figur 4 zeigt den prinzipiellen Aufbau eines Mikromischers 1 mit den Eintrittsöffnungen 15 und 16 für die Zuführung der Arzneistoff-Lösung 11 einerseits und dem Fällungsmittel 12 andererseits, sowie der Austrittsöffnung 17, durch die die im Mikromischer 1 erzeugte Fällungslösung 21 den Mikromischer verlässt. Zwischen den Eintrittsöffnungen 15 und 16 ist die Mikrostruktur 13 angeordnet, mit der die eintretenden Fluidströme 11 und 12 in Einzelströme aufgeteilt werden. Die eigentliche Mischkammer 14 ist oberhalb dieser Mikrostruktur 13 angeordnet. In dieser Mischkammer 14 erfolgt die Durchmischung zu einer homogenen Lösung infolge Diffusion.

In Figur 4a dargestellt ist eine Vergrößerung der zwischen den Eintrittsöffnungen 15 und 16 liegenden Mikrostruktur 13. Die Zuführkanäle 131 und 132 zur Zuführung der Fluidströme in die Mischkammer 14 und zur Unterteilung der eintretenden Fluidströme in Teilströme sind in der Art angeordnet, dass die durch sie aufgetrennten Teilströme ein alternierendes System aus dünnen Fluidlamellen bilden, wobei alternierend ausdrückt, dass Fluidlamellen der Arzneistofflösung 11 und Fluidlamellen des Fällungsmittels 12 abwechselnd geschichtet werden.

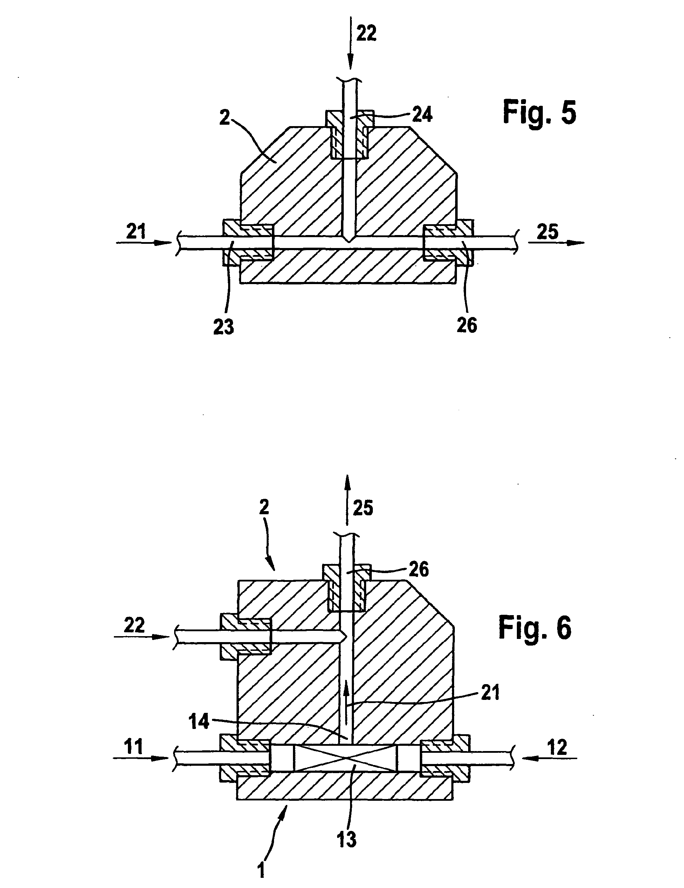

Figur 5 zeigt schematisch den Aufbau eines Segmenters 2 mit den Eintrittsöffnungen 23 und 24 und der Austrittsöffnung 26. Die durch die Eintrittsöffnung 23 dem Segmenter 2 zugeführte Fällungslösung 21 wird mit Hilfe des durch die Eintrittsöffnung 24 zugeführten Transportmediums 22 segmentiert und verlässt den Segmenter 2 als Zweiphasenströmung 25.

Figur 6 zeigt ein zweites Ausführungsbeispiel eines Mikromischers 1 mit einem integrierten Segmenter 2. Die in den Mikromischer 1 eintretenden Fluidströme, die Arzneistoff-Lösung 11 einerseits und das Fällungsmittel 12 andererseits gelangen durch die Mikrostruktur 13 des Mikromischers 1 in die Mischkammer 14 und werden nach Durchmischung in der Mischkammer 14 als weitestgehend homogene Fällungslösung 21 dem Segmenter 2 zugeführt. Im Segmenter 2 wird die Fällungslösung 21 mit einem zweiten, mit der Fällungslösung 21 nicht mischbaren Fluid, das als Trägermedium 22 dient, segmentiert, wobei die dadurch erzeugte Zweiphasenströmung 25 den Segmenter 2 durch die Austrittsöffnung 26 verlässt.

Figur 7 zeigt einen Segmenter 2, der zwei Rohre 27,28 umfasst, von denen das kleinere Rohr 28 in dem größeren Rohr 27 und zu diesem koaxial angeordnet ist. Dabei wird die Fällungslösung 21 mittels des kleinen Rohres 28 in das große Rohr 27 eingeleitet und durch das seitliche durch die Eintrittsöffnung 24 zugeführte Transportmedium 22 segmentiert, so dass eine Zweiphasenströmung 25 den Segmenter 2 am Ende des großes Rohres 27 verlässt.

Figur 8 zeigt ein weiteres Ausführungsbeispiel eines Segmenters 2, bei dem die Zuführkanäle, die die Fällungslösung 21 einerseits und das Transportmedium 22 andererseits zuführen, einen Winkel bilden, der zwischen 0 und 180° variieren kann und bei dem in Figur 8 gezeigten Ausführungsbeispiel 90° beträgt.

Figur 9 zeigt einen Ausschnitt einer rohrförmigen Verweilerstrecke 3 im Querschnitt. Dargestellt ist der Aufbau der der Verweilerstrecke 3 zugeführten Zweiphasenströmung 25. Diese besteht aus vorzugsweise gleichgroßen Fluidvolumen von Fällungslösung 21, die mit Hilfe des Transportmediums 22 segmentiert sind.

Ebenfalls in Figur 9 dargestellt sind die in den Segmenten der Fällungslösung 21 ablaufenden Strömungsvorgänge bzw. die dazugehörigen Strömungsrichtungen. Die über die Zeit gemittelten Geschwindigkeiten ergeben einen über den Rohrquerschnitt der Verweilerstrecke 3 gleichmäßiges Rechteckprofil. Ersichtlich wird, dass sich unter den im Fluidsegment herrschenden Strömungsbedingungen keine Ablagerungen an den Rohrinnenwänden der Verweilerstrecke 3 bilden können.

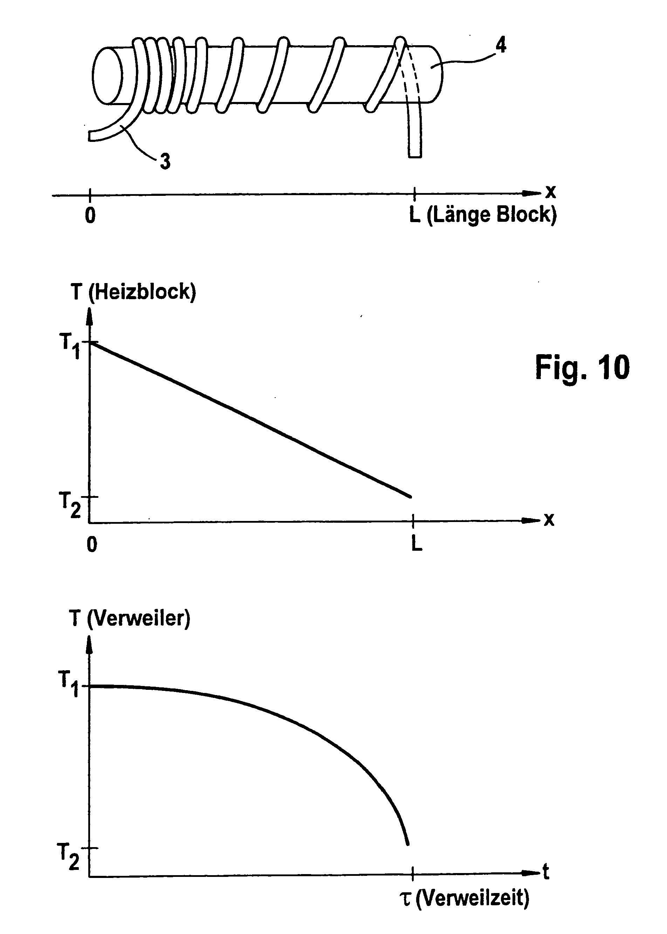

Figur 10 zeigt ein Ausführungsbeispiel einer Verweilerstrecke in der perspektivischen Ansicht, bei der eine schlauchförmige Verweilerstrecke 3 auf ein zylinderförmiges Aluminiumprofil 4 gewickelt ist. Die Verweilerstrecke 3 kann über das Aluminiumprofil 4 gekühlt bzw. geheizt werden.

Wie bereits erwähnt ist es eine Ausführungsform des Verfahrens, in der Verweilerstrecke 3 eine im wesentlichen konstante Übersättigung C₁ (siehe Figur 2 unten) in der Lösung zu realisieren. Dies wird mit einem Temperaturprofil erreicht, bei dem der Temperaturgradient mit der Zeit betragsmäßig zunimmt (siehe Figur 2 oben und Figur 10 unten).

Dieser Temperaturverlauf T(t) wird einerseits durch die Steigung der Verweilerstrecke 3 auf dem Aluminiumblock 4 (siehe Figur 10 oben) und andererseits durch das Temperaturprofil T(L) im Aluminiumblock 4 (siehe Figur 10 Mitte) realisiert, wobei im vorliegenden Beispiel die Temperatur im Aluminiumblock 4 linear abnimmt und die Steigung der auf den Aluminiumblock aufgewickelten Verweilerstrecke 3 zunimmt.

Die Steigung der aufgewickelten Verweilerstrecke 3 und der Temperaturverlauf T(L) im Aluminiumblock 4 sind dem jeweiligen Einzelfall anzupassen. Sie sind abhängig von dem verwendeten Arzneistoff, der Lösung, dem zusätzlichen Eintrag von Festkörperpartikeln als Kristallkeime und dem eventuell eingesetzten Fällungsmittel.

Im folgenden werden für die Wirkstoffe, die Hilfsstoffe, die Lösungs- und Fällungsmittel Beispiele angeführt.

Als Arzneistoffe bzw. Wirkstoffe werden eingesetzt:
- als Anticholinergika: Ipratropiumbromid, Tiotropiumbromid, Tiotropiumbromid-Monohydrat,
- als Betasympathomimetica: Bambuterol, Bitolterol, Carbuterol, Formoterol, Clenbuterol, Fenoterol, Hexoprenalin, Procaterol, Ibuterol, Pirbuterol, Tulobuterol, Reproterol, Salbutamol, Sulfonterol, Terbutalin, Orciprenalin, 1-(2-Fluor-4-hydroxyphenyl)-2-[4-(1-benzimidazolyl)-2-methyl-2-butylamino]ethanol,erythro-5'-Hydroxy-8'-(1-hydroxy-2-isopropylaminobutyl)-2H-1,4-benzoxazin-3-(4H)-on, 1-(4-Amino-3-chlor-5-trifluormethylphenyl)-2-tert.-butylamino)ethano), 1-(4-Ethoxycarbonylamino-3-cyan-5-fluorphenyl)-2-(tert.-butylamino)ethanol,
- als Antiallergika: Dinatriumcromglicat, Nedocromil, Epinastin, und
- als Steroide: Flunisolid, Dexamethason-21-isonicotinat, Seratrodast, Mycophenolate mofetil, Pranlukast, Zileuton, Butixocort, Budesonid, Deflazacort, Fluticason, Proedrol, Mometasin furoat, Tipredan, Beclometason (bzw. das17, 21-Dipropionat), Beclomethason, Douglas, Icomethason enbutat, Ciclometason, Cloprednol, Fluocortin butyl, Halometason, Deflazacort, Alclometason, Ciclometason, Alisactid, Prednicarbat, Hydrocortison-butyratpropionat, Tixocortol-pivalat, Alclometason-dipropionat, Lotrison, Canesten-HC, Deprodon, Fluticason-propionat, Methylprednisolon-Aceponat, Halopredon-acetat, Mometason, Mometasone-furoat, Hydrcortison-aceponat, Mometason, Ulobetasol-propionat, Aminogluethimid, Triamciolon, Hydrcortison, Meprednison, Fluorometholon, Dexamethason, Betamethason, Medryson, Fluclorolon acetonid, Fluocinolon acetonid, Paramethason-acetat, Deprodon Propionat, Aristocort-diacetat, Fluocinonid, Mazipredon, Difluprednat, Betamethason valerat, Dexamethasonisonicotinat, Beclomethason-Dipropionat, Fluocortoloncapronat, Formocortal, Triamcinolon-Hexacetonid, Cloprednol, Formebolon, Clobetason, Endrisone, Flunisolid, Halcinonid, Fluazacort, Clobetasol, Hydrocortison-17-Butyrat, Diflorason, Fluocortin, Amcinonid, Netamethason Diprpionat, Cortivazol, Betamethasonadamantoat, Fluodexan, Trilostan, Budesonid, Clobetason, Demetex, Trimacinolon Benetonid, 9.alpha.chloro-6.alpha.-fluoro-11.beta.17.alpha.-dihydroxy-16.alpha.methyl-3-oxo-1,4-androstadien-17.beta.-carboxysäuremethylester-17-propionat.

Sonstige mit dem erfindungsgemäßen Verfahren hergestellte Arzneistoffe sind Montelukast und Pramipexol.

Als Hilfsstoffe werden für Inhalativa insbesonders Lactose, Glucose, Sucrose, Mannitol, und/oder Trehalose verwendet.

Beispiele für Lösungs- und Fällungsmittel in Abhängigkeit von den herzustellenden Wirkstoffen zeigen die folgenden Tabellen, wobei Lösungs- und Fällungsmittel mischbar sein müssen.

Für Anticholinergikal/Betasympathomimetica/Antiallergika:

| Wirkstoffe | Lösungsmittel | Fällungsmittel |
|---|---|---|
| Salzformen | Wasser, Methanol | Alkohole (Ethanol, Propanol, iso-Propanol), Ketone (Aceton, Butanon) |
| Freie Basen | Alkohole (Ethanol, Propanol, iso-Propanol, tert.-Butanol), Ketone (Aceton, Butanon) | Wasser, Methanol |

Für Steroide:

| Wirkstoffe | Lösungsmittel | Fällungsmittel |
|---|---|---|
| Polare | Ketone (Aceton, Butanon) | Alkohole (Methanol, Ethanol) |
| | Alkohole (Ethanol, Propanol, iso-Propanol, tert.-Butanol), Ketone (Aceton, Butanon) | Wasser, Methanol |
| | Aromaten (Toluol, Ethylbenzol) | Alkohole (Ethanol, Propanol, iso-Propanol) |
| Unpolare | Halogenkohlenwasserstoffe (Dichlormethan, Trichlormethan | Alkohole (Ethanol, Propanol, iso-Propanol), Ether (Dimethylether, Dioxan) |

Beispiele für Transportmedien in Abhängigkeit von den herzustellenden Wirkstoffen und den verwendeten Lösungsmitteln zeigen die folgenden Tabellen, wobei Lösungsmittel und Transportmedien nicht mischbar sind.

| Wirkstoffe | Lösungsmittel | Transportmedien |
|---|---|---|
| Polare | Wasser, Alkohole (Methanol, Ethanol, Propanol, iso-Propanol, tert.-Butanol), Ketone (Aceton, Butanon) | Flüssigkeiten: Kohlenwasserstoffe (Benzine, Petrolether, Cyclohexan, Decalin, Benzol, Toluol, Xylole) Gase: Luft, Stickstoff, Kohlendioxid, Helium, Argon |
| Unpolare | Halogenkohlenwasserstoffe (Dichlormethan, Trichlormethan), Ether (Diethylether, Dibutylether), Aromaten (Toluol, Ethylbenzol) | Flüssigkeiten Wasser, Alkohole (Methanol), Amide (Formamid) Gase: Luft, Stickstoff, Kohlendioxid, Helium, Argon |

### Bezugszeichenliste

- 1: Mikromischer
- 2: Segmenter
- 3: Verweilerstrecke
- 4: Aluminiumprofil
- 11: Arzneistoff-Lösung
- 12: Fällungsmittel
- 13: Mikrostruktur
- 14: Mischkammer
- 15: Eintrittsöffnung
- 16: Eintrittsöffnung
- 17: Austrittsöffnung
- 21: Fällungslösung
- 22: Transportmedium
- 23: Eintrittsöffnung
- 24: Eintrittsöffnung
- 25: Zweiphasenströmung
- 26: Austrittsöffnung
- 27: Großes Rohr
- 28: Kleines Rohr
- 131: Zuführungskanäle
- 132: Zuführungskanäle

## Patentansprüche

1. Mikroreaktor zur Durchführung eines Verfahrens zur kontinuierlichen Herstellung inhalierfähiger Arzneistoffe, bei dem eine Arzneistoff-Lösung (11) eingesetzt wird, die Lösung mittels eines Segmenters und eines Transportmediums (22) segmentiert wird, der Kristallisationsprozess in einer Verweilerstrecke (3) mittels Aufprägen einer definierten Temperatur eingeleitet und geführt wird, wobei zunächst mittels einer sprunghaften Temperaturverringerung der Keimbildungsprozess in der Art eingeleitet wird, dass die Lösung bzgl. der Temperatur T und der Konzentration C des in ihr gelösten Stoffes einen übersättigten, metastabilen oder labilen Zustand annimmt, und anschließend das Kristallwachstum durch gezielte Kühlung beeinflusst wird, und Kristallpartikel von den übrigen Phasen nach Durchlauf der Verweilerstrecke in einem Abscheider abgeschieden werden, **dadurch gekennzeichnet, dass**
- die Dimensionen des Mikromischers zur Aufteilung der zugeführten, zu mischenden Fluide im Bereich von 10µm bis 1 mm, vorzugsweise zwischen 25µm bis 200 µm, liegen,
- die Dimensionen der Kanäle des Segmenters im Bereich von 0,1 bis 5 mm, vorzugsweise im Bereich zwischen 0,2 mm und 5 mm liegen, und
- die Verweilerstrecke schlauch-, rohr- oder kanalförmig ausgebildet ist mit Durchmessern ihrer Kanäle im Bereich von 0,5 bis 10 mm, vorzugsweise 1 mm bis 2 mm, und eine Länge aufweist zwischen 10 cm und 200 m, vorzugsweise zwischen 1 m und 25 m.

2. Inhalierfähiger Arzneistoff mit einem aerodynamischen Durchmesser kleiner 20µm, vorzugsweise kleiner 5µm und größer 0,3µm, **dadurch gekennzeichnet, dass** er mittels einer Vorrichtung nach Anspruch 1 hergestellt wird.
